Europäisches Patentamt

European Patent Office

Office européen des brevets

(11) Publication number: **0 305 145 A2**

## EUROPEAN PATENT APPLICATION

(21) Application number: 88307793.5

(22) Date of filing: 23.08.88

(51) Int. Cl.⁴: **C12Q 1/68** , //C07H21/02

(30) Priority: 24.08.87 US 88737

(43) Date of publication of application:
01.03.89 Bulletin 89/09

(84) Designated Contracting States:
BE DE ES FR GB IT LU NL

(71) Applicant: ORTHO DIAGNOSTIC SYSTEMS INC.
U.S. Route 202
Raritan New Jersey 08869(US)

(72) Inventor: Zivin, Robert A.
16 Fieldstone Place
Flemington, N.J. 08822(US)
Inventor: Monahan, John A.
23 Harding Road
North Reading, Mass. 01864(US)

(74) Representative: Jones, Alan John et al
CARPMAELS & RANSFORD 43 Bloomsbury Square
London, WC1A 2RA(GB)

(54) **Methods and probes for detecting nucleic acids.**

(57) An in solution hybridization method utilizes two soluble oligonucletide probes. One of the probes has a homopolynucleotide tail which is captured by a solid support coated with a complementary homopolynucleotide. A ribosomal RNA sequence specific to M. pneumoniae and synthetic probes complementary thereto are provided. Methods are provided for specifically identifying the presence of Mycoplasma pneumoniae 16S ribosomal RNA in a sample by using at least one synthetic nucleic acid probe complementary to the 16S ribosomal RNA in a hybridization assay. The methods comprise providing at least one synthetic probe which has a sequence complementary to at least a portion of a ribosomal RNA sequence specific to M. pneumoniae.

## METHODS AND PROBES FOR DETECTING NUCLEIC ACIDS

### Background of the Invention

Throughout this application, various publications and patents are referenced. The disclosures of these publications and patents in their entireties are hereby incorporated by reference into this application in order to more fully describe the state of the art as known to those skilled therein as of the date of the invention described and claimed herein.

This invention relates to an in solution hybridization method for detecting nucleic acids which utilizes two specific and soluble oligonucleotide probes.

The present invention also relates to RNA sequences and probes complementary thereto which are specific for Mycoplasma pneumoniae. The probes may be used in methods for specifically detecting Mycoplasma pneumoniae in a sample with limited or no cross-reaction from other Mycoplasma species.

Nucleic acid hybridization procedures may involve one of two formats, namely in solution hybridization and immobilized hybridization. In in solution hybridization, the probes and sample nucleic acids are free in solution during hybridization. In immobilized hybridization, either the sample or a probe may be immobilized prior to hybridization with hybridization taking place in the insoluble phase. One method for carrying out in solution hybridization involves using hydroxyapatite for selectively binding hybridized DNA. Other methods for in solution hybridization are described in International Patent Application Publication No. WO 84/02721. An example of immobilized hybridization is set forth in U.S. Patent No. 4,358,535 (Falkow et al.).

In the present method a target nucleic acid sequence is permitted to hybridize in solution with two specific and soluble oligonucleotide probes, wherein one of the probes has a homopolynucleotide tail. Only after the hybridization is complete is the sample containing the hybridized complex brought into contact with and captured by an insoluble solid support. The capture technique described herein utilizes complementary homopolynucleotide tails on the solid support and the capture oligonucleotide.

The use of polyuridylic acid (poly U) coated paper to capture polyadenylic (poly A) containing mRNA is described in Wreschner, D.H. et al., Nucleic Acids Research, 12: 1349-1359 (1984). A hybridization method using a polydeoxyguanylic acid (Poly dG) cellulose support to capture a polycytidylic acid (poly C) probe is described in Vary, C.P.H. et al, Clin. Chem. 32(9):1696-1701 (1986). The method described by Vary et al. involves a competitive hybridization assay in which a signal nucleic acid strand is displaced by a target strand. The signal nucleic acid strand used is not complementary to the target nucleic acid and there is no formation of a ternary hybridization complex. Thus, the method described does not involve a sandwich hybridization assay. Furthermore, the detection method described involves detecting a label in solution rather than on an insolubilized solid support.

The present method involves a sandwich hybridization assay which takes place in solution. A ternary or tripartite hybridization complex is formed in solution and target DNA is detected by detecting a label or reporter molecule on a solid support. The present method is useful in detecting any type of nucleic acid containing organism. In particular, the present method is useful for detecting Mycoplasma pneumoniae.

The genus Mycoplasma encompasses a diverse group of organisms that represent the smallest free living procaryotes, having a genome size of approximately $5 \times 10^8$ daltons. Because of their small size, Mycoplasmas are very difficult to identify as the fully developed colonies are too small for identification with the unaided eye.

The only Mycoplasma species proven to cause a human disease is M. pneumoniae, which is the etiological agent of atypical pneumonia, commonly referred to as "walking pneumonia"; a disease usually not fatal but difficult to diagnose. It is desirable to diagnose M. pneumoniae as quickly as possible in order to administer the proper antibiotic therapy in the early stages of the disease.

The use of DNA probes for the detection of microbes is well established, but suffers from a lack of sensitivity unless lengthy sequences of bases are used as targets. Such methods necessitate the use of DNA probes which are very large, i.e. over 500 bases in length. This is a disadvantage because large DNA probes are not suitable to being efficiently produced by synthetic methods and must be prepared by recombinant DNA techniques which are costly and time consuming. A further disadvantage is that large probes lack specificity because they are more likely to have nucleic acid sequences which are complementary to the sequences of many different nucleic acids. Finally, probes produced by recombinant techniques must be isolated and purified from a complex biological mixture containing many different nucleic acids. Such procedures are tedious and result in low yields of probes which may not be pure.

Methods for detecting various species of Mycoplasma by hybridization assays which utilize DNA probes complementary to Mycoplasma ribosomal RNA sequences have been described. For example see: Gbel U.B. et al. (1984) Science 226:1211-1213; and Razin S. et al., (1984) Isr J. Med. Sci 20:758-761. These methods utilize a single genomic probe of about 1000 or more bases. Such a large genomic probe is difficult to synthesize synthetically and generally must be produced by cloning in an E. coli host cell. Additionally, these methods are not specific for any one Mycoplasma species and are therefore, complicated by interspecies cross-reaction problems.

It is an object of the present invention to avoid reliance upon recombinant DNA techniques by providing probes which are suitable to being chemically synthesized.

Methods for detecting microorganisms in general by use of DNA probes complementary to ribosomal RNA contained in the organism are taught in International Patent Application Publication No. WO 84/02721. These methods utilize a single large DNA probe. WO 84/02721 describes an in solution hybridization method which uses a probe complementary to a particular fraction of an rRNA sequence to detect the rRNA of a single group of organisms, thereby detecting the presence of one or more organisms characteristic of that group. However, the method taught is not species specific.

Species specific detection of Mycoplasma hyorhins using DNA probes was described in Taylor, M.A. et al., (1984) Isr. J. Med. Sci. 20:778-780. This method utilized a genomic DNA fragment as a single DNA probe complementary to a conserved sequence of ribosomal RNA. The smallest probe described contained over 3000 bases.

DNA probes specific for M. pneumoniae and M. genitalium rRNA have been reported by Razin, S. et al. in Abstracts of the Sixth International Congress of the IOM, August 26-31, 1986, Birmingham, Alabama, p. 81. The probes used were selected from genomic libraries. Gobel, U. et al. reported using synthetic oligonucleotides of 20-30 bases in length which reacted with both M. pneumoniae and M. genitalium (supra, p. 82).

An object of the present invention is to provide oligonucleotide probes specific for M. pneumoniae. Hybridization methods specific for M. pneumoniae are provided which utilize at least one probe complementary to a specific M. pneumoniae ribosomal RNA sequence. The probes are of a size which makes them suitable to being prepared by synthetic methods.

A method for identifying microbial nucleic acids by a one-step sandwich hybridization assay is described in U.S. Patent Nos. 4,563,419 and 4,486,539 (both in the names of Ranki et al.). The method generally involves hybridization of a target single-stranded microbial nucleic acid with two different complementary nucleic acid probes, one of which is attached to a solid support and the other having a detectable label. Similar methods are also described in Virtanen, M. et al., (1983) Lancet 1 - (8321):381-383; and Ranki, M. et al. (1983) Gene 21:77-85. These methods have the disadvantage of utilizing large probes which must be prepared by recombinant DNA techniques and they are not species specific.

The present invention provides an improved one-step sandwich hybridization assay, specific for M. pneumoniae, which utilizes synthetic oligonucleotide probes.

## Summary of the Invention

The present invention provides a method for determining the presence or amount of a target nucleic acid sequence in a sample suspected of containing the target sequence. In order to carry out the method, soluble oligonucleotide probes are provided which are complementary to different portions of the target nucleic acid sequence. A soluble capture oligonucleotide is provided which has a homopolynucleotide tail covalently attached thereto. Additionally provided is a soluble detectable oligonucleotide probe which is complementary to a substantially different portion of the target sequence than the capture oligonucleotide. The capture oligonucleotide, detectable oligonucleotide and single stranded target nucleic acid are mixed in an aqueous solution under hybridization conditions so as to form a soluble tripartite hybridization complex. The tripartite complex is then contacted with an insoluble solid support having homopolynucleotide tails covalently attached thereto. The immobilized homopolynucleotide tails are complementary to the tail on the capture oligonucleotide. The tail on the capture oligonucleotide hybridizes with the immobilized tail thereby immobilizing the tripartite complex. The detectable oligonucleotide is then detected or measured on the solid support.

The present invention also provides a 16S rRNA sequence which is specific for M. pneumoniae and nucleic acid probes complementary to the RNA sequence.

Additionally, the present invention provides improved methods for identifying the presence of Mycoplasma pneumoniae ribosomal RNA in a sample in a hybridization assay. The methods utilize at least one synthetic nucleic acid probe complementary to the M. pneumoniae 16S rRNA, wherein each sequence is complementary to at least a portion of the rRNA sequence of the present invention.

The present method for specifically detecting M. pneumoniae relies upon the discovery of a specific sequence of bases of the 16S rRNA of M.pneumoniae. A 115 base sequence is provided which is specific for M. pneumoniae and M. genitalium. When this sequence is used as a target for one or more oligonucleotide probes in a hybridization assay, a high degree of specificity and sensitivity is achieved which is not present in prior methods. The prior methods do not teach how to distinguish between the various species of Mycoplasma. Prior methods have many shortcomings including reliance on large non-specific probes and much cross-reaction between species. The present invention avoids these shortcomings through use of short synthetic probes, some of which are specific for M. pneumoniae, which reduce or eliminate cross-reaction with other Mycoplasma species.

Brief Description of the Figures

Figure 1 depicts an 80 base sequence (which is part of the 115 base sequence of the present invention) and the sequences of the complementary probes ODS 55 and ODS 56.

Figure 2 depicts the 300 base sequence of the 5′end of the 16S rRNA of M. pneumoniae, designated MPRDNAI.

Detailed Description of the Invention

The in solution hybridization method of the present invention may be qualitative or quantitative. That is, it may be used to merely detect the presence of a target nucleic acid in a sample or it may be used to determine the amount of the target nucleic acid in the sample. The sample tested can be derived from any biological material, such as a biological fluid including blood, plasma, sputum, urine, mucous, cerebrospinal fluid; any environmental material, such as water or soil; and any material derived from food.

The target nucleic acid sequence can be from any organisms containing nucleic acid, i.e. DNA or RNA, such as bacteria, including pathogens; viruses; fungi, such as yeasts; and protozoans. The target nucleic acid sequence may be either DNA or RNA and it is characteristic of the specific organism from which it is derived. Thus, detection of the specific nucleic acid sequence corresponds to detection of the organism in the sample tested.

The capture and detectable oligonucleotide probes of the present invention are generally between 11 nucleotides and about 100 nucleotides long. The probe should generally be at least 11 nucleotides long in order to allow for efficient hybridization with complementary sequences in the target nucleic acid. It is not necessary for the entire probe sequence to hybridize with the target sequence, as long as, generally, at least 11 consecutive nucleotide residues in the probe hybridize. Thus, the two probes may be overlapping in certain portions and a successful sandwich assay may still result. However, it is preferred that the probes are complementary to adjacent target sequences which either abut or are separated by one or more nucleotides, thus allowing substantially all of the probe to hybridize with the target sequence. Hybridization refers to complementary base pairing.

The homopolynucleotides used in the present method are homopolymers of single nucleotides. Suitable homopolynucleotides include polyuridylic acid (poly-U), polyadenylic acid (poly-A), polythymidylic acid (poly-T), polyguanylic acid (poly G), and polycytidylic acid (poly C). Also, the corresponding deoxyribonucleotides may also be used, e.g. polydeoxyadenylic acid in place of polyadenylic acid. The tails on the capture oligonucleotide and the solid support are generally at least about 50 nucleotides long and up to about 5000 nucleotides long, preferably in the range 100-1000 nucleotides long. The homopolynucleotide tail on the capture oligonucleotide is complementary to the homopolynucleotide tail on the solid support. Preferably, the tail on the capture oligonucleotide is poly A and the tail on the solid support is poly U. Tailing or tails refers to the addition of a sequence of nucleotides to the end of a nucleic acid sequence. The tails may be added to the 3′ end of the oligonucleotide probe end, using the enzyme terminal transferase. The tail may also be added at the 5′ end by suitable chemical or enzymatic means, such as using the enzyme DNA ligase. Preferably, the tail is added at the 3′ end. Tailing procedures using the enzyme terminal transferase are well known in the nucleic acid hybridization art.

Before hybridization can occur, the target nucleic acid sequence must be removed from the organism containing the sequence and the sequence must be provided in single stranded form. The target sequence can be removed from the organism containing it by lysis of the organism with a suitable detergent or proteolytic and/or hydrolytic enzyme. The target nucleic acid from the lysed cells must be in single stranded from and this can be achieved by denaturing the DNA using standard techniques, such as exposure to heat, extremes of pH, decrease in the dielectric constant of the aqueous medium or exposure to urea, amides or similar solutes. Denaturation is the separation of complementary strands of double-stranded nucleic acid. All or part of the nucleic acid molecule may be denatured, as long as an amount sufficient to allow hybridization is provided. If the nucleic acid is already in substantially single stranded form, such as single stranded ribosomal, messenger and transfer RNAs, then a specific denaturation step is not required. The lysis/denaturation solution used may contain the capture and detectable oligonucleotides. The sample containing the target nucleic acid may then be conveniently added at the site of sample collection and then stored or assayed immediately. The only further steps required would be contacting with the insolubilized support and washing to remove unhybridized material.

In order for hybridization to occur the probes and target sequence must be incubated at a specific temperature for a period of time to enable the tripartite hybridization complex to form. The length of time required for hybridization varies with the conditions used such as the concentration of the probes, the concentration of the target nucleic acid sequence and the rate of hybridization which is characteristic of the conditions used. The conditions necessary for achieving hybridization of nucleic acid sequences are set forth in Maniatis, T. et al. Molecular Cloning: A Laboratory Manual, Cold Spring Harbor Publication (1982).

The detectable oligonucleotide used in the method of the present invention may be detectable by virtue of a reporter molecule being covalently attached to the molecule. The reporter molecule may be any molecule which is capable of being detected such as a radioactive molecule, fluorescent molecule or chemiluminescent molecule. The reporter molecule may be attached directly to the complementary oligonucleotide or may be attached to the oligonucleotide via a polynucleotide tail which is in turn covalently attached to the detectable oligonucleotide. The detectable oligonucleotide may also be detectable by virtue of a specific ligand covalently coupled to the molecule. Suitable ligands are biotin, enzymes capable of producing a detectable color change, an antibody-specific antigen or an antigen-specific antibody. The ligand may be directly coupled to the detectable oligonucleotide or may be covalently attached via a polynucleotide tail which is covalently attached to the oligonucleotide. The ligand may also be attached via a chemical spacer arm. The polynucleotide tail may be a homopolymer or homocopolymer, i.e. a nucleic acid chain with only two types of nucleotides in alternating or random sequence, such as adenine and uracil or guanine and thymine.

The insoluble solid support used to capture the tripartite complex may be any silicaceous, polysaccharide or plastic material. The solid support is coated with homopolynucleotide tails, which are covalently attached to the support. The solid support may take the form of beads, microparticles, macroparticles, microtiter wells, gels or filter papers. In a preferred emobodiment, the solid support is made of a polysaccharide material, such as a cellulosic material, and preferably in the form of a filter paper. Filtration of the soluble tripartite complex through the filter paper solid support provides a convenient means for insolubilized the tripartite complex as well as separating the complex from unhybridized ingredients. The insolubilized tripartite complex can then be conveniently washed on the filter.

Thepresent invention provides nucleic acid probes which are complementary to the following 115 base nucleotide sequence of the M. pneumoniae 16S rRNA:

5′ ACCGCAUAAGAACUUUGGUUCGCAUGAA UCAAAGUUGAAAGGACCUGCAAGGGUUCGUUAU UUGAUGAGGGUGCGCCAUAUCAGCUAGUUGGUG GGGUAACGGCCUACCAAGGCA 3′

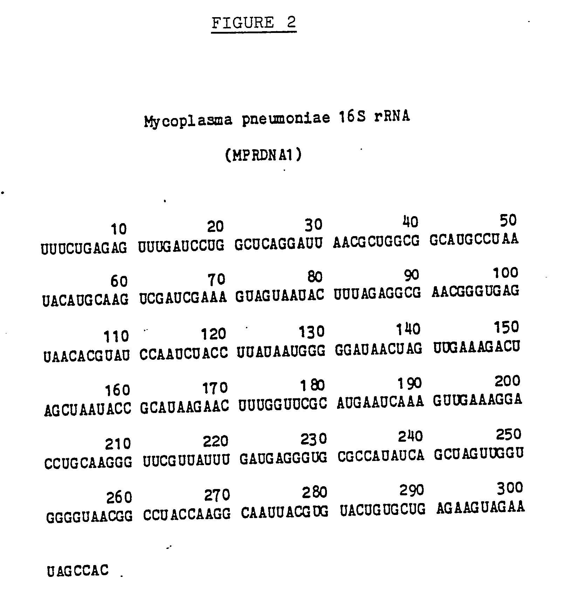

wherein A, C, G and U represent the bases adenine, cytosine, guanine and uracil, respectively. Additionally, probe sequences which extend beyond the 115 base sequence into other regions of the 300 base sequence of Figure 2 (i.e, bases 1-157 and 273-300) are considered within the scope of the present invention. Any fragment of from, generally, about 10 to about 115 bases of the 115 base RNA sequence is also within the scope of the present invention. The present invention also provides the DNA or transcriptional equivalent of the 115 base RNA sequence, wherein a thymine base (T) is substituted for each uracil base. The transcriptional equivalent corresponds to the DNA from which the 16S rRNA is transcribed. It is contemplated that the rRNA sequence of the 115 base sequence, or fragments thereof, may itself be used as a nucleic acid probe which is complementary to the DNA strand which transcribes the 16S rRNA,i.e. the coding strand of the duplex DNA.

Nucleic acid sequences which are complementary to at least a portion of the 115 base sequence are useful as oligonucleotide probes for detecting M. pneumoniae. Two such probes are ODS 55 (a 33 mer) and ODS 56 (a 37 mer), represented as:

ODS55:

3′ GTATTCTTGAAACCAAGCGTACTTA GTTTCAAC 5′,

and

ODS56:

3′ CTGGACGTTCCCAAGCAATAAACTA CTCCCACGCGGT 5′.

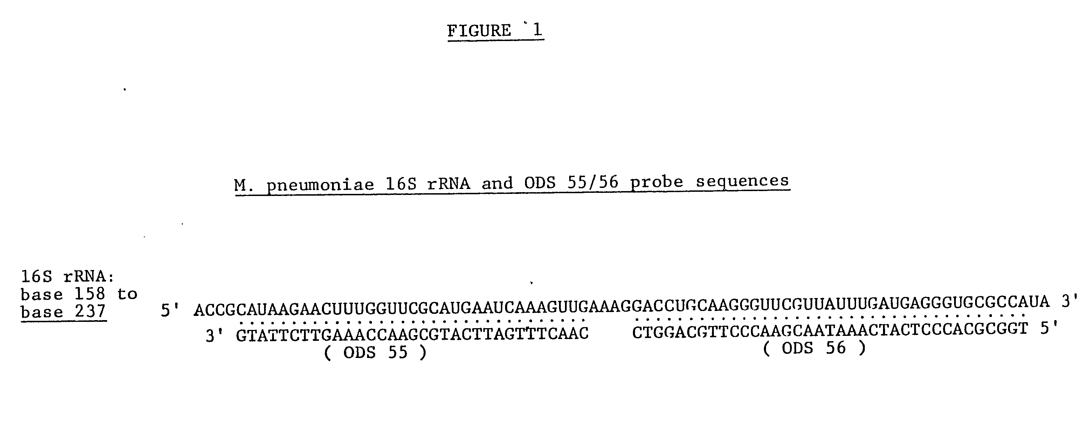

The complementary relationship between the 16S rRNA sequence and ODS 55 and ODS 56 is depicted in Figure 1.

Additionally, the oligonucleotide probes of the present invention may have the sequences of ODS-59 (a 26 mer), ODS-60 (a 26 mer) or ODS-81 (a 37 mer): which are each complementary to the 115 base sequence, represented as:

ODS-59:

3′ CCAAGCGTACTTAGTTTCAACTTTCC 5′.

ODS-60:

3′TGGACGTTCCCAAGCAATAAACTACT 5′ and

ODS-81:

3′ ATAGTCGATCAACCACCCATTGCCG-GATGGTTCCGT 5′.

Under hybridization conditions of proper stringency, ODS-55, ODS-59 and ODS-81 were determined to be specific for M. pneumoniae with no cross-reaction with M. genitalium. The probes ODS-56 and ODS-60 reacted with M. genitalium as well as M. pneumoniae.

The probes of the present invention generally have at least ten bases in order to ensure efficient hybridization. The complementary portion of the probes may be up to about 50 bases in length. The probes may also have additional noncomplementary base sequences covalently coupled thereto, e.g. by "tailing". The "tailed" regions may be used for attaching detectable labels or solid supports. The probes may have one or more detectable labels attached. Additionally, the probes may be immobilizable (e.g. tagged with biotin or some other hapten which can in turn be subsequently captured by a solid support) or immobilized to a solid support.

The probes of the present invention may be used in a method for identifying the presence of M. pneumoniae 16S ribosomal RNA in a sample. In such a method, at least one of the probes complementary to the ribosomal RNA may be used in a hybridization assay. The probe is preferably complementary to at least a portion of the 115 base RNA sequence of the present invention.

The probes of the present invention may also be used in a sandwich hybridization assay for identifying the presence of M. pneumoniae 16S ribosomal RNA in a sample. In the sandwich format, two oligonucleotide probes of the present invention, complementary to different portions of the ribosomal RNA, are utilized in a one-step hybridization assay. Each probe has a sequence which is complementary to at least a portion of the 115 base RNA sequence. The combination of ODS55/ODS81 or ODS59/ODS81 may be useful in a dual probe assay because each of these probes reacts specifically with M. pneumoniae and the combinations are non-overlapping.

In the sandwich assay format, the probes may be either overlapping or non-overlapping. It is preferred that the probes be non-overlapping, i.e. no overlap of complementary base sequences, in order to maximize hybridization. If non-overlapping, the probe sequences are adjacent to each other along the target rRNA sequence and the probes may either abut or be separated by one or more bases, as depicted in Figure 1. If the probes are overlapping, i.e. they are complementary to at least a portion of the same sequence of bases on the 16S rRNA, the probes can still be used in the sandwich assay, but hybridization will not be maximized.

The advantage of a sandwich assay system is its ability to provide maximum specificity by relying upon both probes, since individually the probes might cross-react with other organisms present within a test sample. Such cross-reactivity is advantageously avoided by relying upon both probes to interact with a target molecule in order to allow its detection. Thus, if only one of the probes non-specifically reacts with a target by virtue of cross-reactivity, no signal will be obtained since the other probe will not also cross-react with the same target. In this manner the specificity of the test will be preserved.

In the immobilization-type hybridization assay, the actual mechanism of the sandwich assay comprises one probe being immobilized to a solid support and the other probe labelled with a detectable label or "reporter" substance (or tagged with a ligand to which the reporter can specifically bind). Only the combined binding of both the "immobile" and "reporter" probes yields a positive signal.

The detectable label or reporter molecule utilized may be any molecule which is capable of being detected. Examples of such detectable molecules are radioisotopes, e.g. $^{32}P$, fluorescent molecules, e.g. FITC, enzymes which produce color changes in dyes, e.g. horse radish peroxidase, light scattering particles and chemiluminescent catalysts. Another suitable label is a ligand capable of binding to specific proteins which have been tagged with an enzyme, fluorescent molecule or other detectable molecule. An example of such a suitable ligand is biotin, which will bind to avidin or streptavidin.

The solid support to which the probes of the present invention may be immobilized may be any silicaceous, polysaccharide or polymeric material. Suitable solid supports may be made of polysaccharide materials, such as cellulose and agarose, or polymeric materials, such as latex. Additionally, microporous surfaces, such as nitrocellulose paper, as well as glass fiber filters and controlled pore glass may be used. In one embodiment, agarose beads which are coated with streptavidin are used to immobilize a biotinylated probe. In another embodiment agarose beads may have biotinylated oligonucleotides attached and the coated beads may be reacted with a streptavidin-horseradish peroxidase complex.

The probes of the present invention are made synthetically rather than being derived from genomic DNA or RNA. Methods of chemically synthesizing the oligonucleotide probes of the present invention are well known in the art. One such method is the phosphoramidite method described in U.S. Patent No. 4,458,066 (Caruthers et al). Other methods are described in V. Amarnath et al. (1977), Chem. Rev. 77: 183-217. The preferred probes of the present invention are relatively short, e.g. 20-50 nucleotide oligomers complementary to the 115 base sequences as described herein, and therefore can be efficiently made by an automated DNA synthesizer. The probes may be tailed with additional nucleotides, for example biotin labeled nucleotides, of up to 5000 bases. Chemical synthesis of the probes makes it possible to easily produce large numbers of purified probes with specific nucleotide sequences rather than relying on the difficult recombinant procedures of isolating and purifying the genetic information from a natural source.

In order to prepare the probes of the present invention, a partial sequence of the M. pneumoniae 16S rRNA was obtained and the sequence confirmed. Methods of determining 16S rRNA sequences are well known in the art, as exemplified in Lane, D.L. et al. (Oct. 1985) Proc. Natl. Acad. Sci., 82:6955-6959. Specifically, a 300 base sequence at the 5'end of the 16S rRNA was obtained and the 115 base sequence confirmed. This sequence, designated MPRDNA1, is set forth in Figure 2.

In order to determine the 115 base sequence, the MPRDNA1 sequence was utilized in a sequence homology analysis with other known 16S rRNA sequences of closely related bacterial species. It is known that there is little detectable base homology between the Mycoplasma species and that M. pneumoniae appears to be unrelated to any of the other Mycoplasma except M. genitalium - (Sugino, W.M. et al (1980) J. Gen. Micro. 121:333-338). In fact, M. pneumoniae shares a high degree of homology with M. genitalium. However, M. pneumoniae is less closely related to gram positive bacteria less than it is to some other Mycoplasmas. Therefore, the 16S rRNA sequence of a gram positive bacterium, B. subtilis, was used in the analysis, as well as the reported 16S rRNA sequence of another Mycoplasma species, M. capricolum. The 16S rRNA sequence of E. coli, a gram negative bacteria which is phylogenetically distant from M. pneumoniae, was used as a reference sequence.

The M. capricolum sequence was a 1992 bp ds-DNA sequence as determined by Iwami, M. et al. (1984), Mol. Gen. Genet. 196: 317-322. The B. subtilis sequence was a 1172 bp dS-DNA sequence as determined by Stewart, G.D. et al. (1983), Nucl. Acid Res. 11:6289-6300. The E. coli sequence was a 1541 bp rRNA sequence as determined by Ehresmann, C. et al. (1977), FEBS Lett 84:337-341; Carbon et al. (1978), FEBS Lett. 94:152-156; and Carbon et al. (1979) Eur. J. Biochem. 100:399-410.

The following examples are presented to illustrate the subject invention. The invention is not to be considered limited by these examples but only by the appended claims.

Example 1

Dot Blot Hybridization Assay Using a Single Probe

Clinical samples of M. pneumoniae were taken as throat swabs from infected patients. 215 samples were tested by a simple dot blot hybridization assay using $^{32}$P-labelled ODS-56. The results of the assay indicated that five samples were M. pneumoniae. These five positive samples were confirmed by culture to be M. pneumoniae. The methodology of this assay is as follows:

The 16S rRNA present in the cells isolated by the throat swabs was extracted with phenol after the liquid from each swab, including mucus, had been centrifugally eluted from the swab prior to phenol extraction. The eluted liquid from each swab was mixed with 100 microliters of phenol saturated with approximately 0.1 M Hepes, pH 7.4. The mixture was vortexed, spun in a microfuge and the supernatant removed. This procedure was repeated one more time on the supernatant.

The supernatant was mixed with 1/10 volume of 5 M NaCl, vortexed, microfuged and the supernatant removed leaving behind a pellet. The supernatant was extracted two times with ether. The ether was removed, leaving the aqueous phase in the tube. This remaining lower phase was dried in a centrifugal evaporator to remove the remaining ether.

An aqueous buffer designated SSPE was used in subsequent steps. SSPE (20x) was prepared in a liter volume by adding 174g NaCl, 27.6g $NaH_2PO_4 \cdot H_2O$, 7.4g EDTA and the pH adjusted to 7.4 with NaOH. Another aqueous solution utilized, designated F/S, was 37% formaldehyde: SSPE, 20:30 in volume ratios.

The residue remaining after the extraction steps was resuspended in 100 microliters of F/S, vortexed and heated at 60°C for 15 minutes. At room temperature, 200 microliters of 10x SSPE was mixed and filtered through a pre-wetted "Gene Screen+" (New England Nuclear) nylon membrane (pre-wetted with $H_2O$, then 20x SSPE) using a 96 well dot-blotter (Schleicher and Schuell). The samples were permitted to bind to the filter and then the fluid was sucked through with vacuum. Each well was washed with 100 microliters 10x SSPE and dried 1 hr at 80°C in a vacuum oven.

After drying, the filters were contacted for 0.5 hr. at 37°C with a prehybridization solution consisting of 25% formamide, 4x SSPE, 0.2% SDS, 1x Denhardt's solution and 100 micrograms/ml tRNA. Then, the filters were contacted with the same mixture containing $^{32}$P-labelled ODS 56 (3 pmoles;50 microliters in 5 ml) and allowed to incubate at 37°C for 1 hr. The blots were washed exposed to X-ray film. Five positive dots resulted indicating that ODS 56 is capable of identifying M. pneumoniae 16S rRNA from a clinical sample.

## Example 2

### Sandwich Hybridization Assay

The general principle of the sandwich assay utilized is described briefly as follows:

Two oligonucleotides, complementary to adjacent regions of the M.pneumoniae 16S rRNA, were tailed to create a capture and detection oligonucleotide system. ODS-56 was tailed with approximately 100-200 residues of adenylic acid (poly A) for the purpose of creating a capture oligonucleotide for capturing the RNA and immobilizing it. The ODS-81 was tailed with a mixture of dCTP and biotin dUTP (5:1) for use as the detection oligonucleotide. The capture of the rRNA was carried out by passing a hybridized oligo/RNA complex through a paper filter ("Hybond"-mAP messenger affinity paper; Amersham) to which poly U tails had been attached. The messenger affinity paper specifically binds, in a reversible manner, poly A containing molecules. Thus, the poly A tails on ODS-56 hybridized to the poly U tails on the paper, thereby immobilizing the complex. Streptavidin-horseradish peroxidase (HRP) (Sigma; 1/1000 dilution of 0.5 mg/ml, #S-5512) was passed through the filter, bound to the immobilized biotin (on ODS-81) and after several dodecyl sulfate (SDS) washes (and a citrate/Tween® final wash) was detected by 3,3',5,5'-tetramethyl benzidine (TMB) staining. TMB is a chromogen for HRP.

The probe ODS-81 was biotin tailed at its 3'-end using the enzyme terminal transferase and a nucleotide mixture containing about 20% biotinylated dUTP (Bethesda Research Labs, Biotin-11-UTP) and the rest dCTP. 14 microliters of ODS-81 (250 picomoles) was added to 258 microliters of water. Then, 40 microliters of 10X TdT buffer was added along with 4 microliters of dCTP (40 nmoles), 31 microliters biotinylated dUTP (12.4 nmoles) and 60 microliters of terminal transferase (Pharmacia, FPLC pure). This mixture was incubated for 4 hours at 37°C and purified with a G-50 Sephadex®column. ODS-56 was similarly tailed using dATP (Pharmacia).

The M. pneumoniae rRNA utilized was obtained from lab cultures of M. pneumoniae. The sandwich assay was run using either rRNA extracted from the cells or a lysed cell mixture containing the rRNA. Cultures of M.pneumoniae (in the late log phase) were lysed by taking two T25 flasks containing the cultures and adding 1ml of Hepes buffered 1% SDS and 25mM DTT after removing the growth media. The resulting mixture was then diluted with 50mM Pipes, pH 6.58 and 0.1% SDS to obtain approximately $10^{11}$ cell equivalents/ml.

The actual steps utilized were as follows:
A sample, containing approximately 0.5% lithium dodecyl sulfate, 10mM EDTA, 25 mM dithiothreitol (DTT), 50 mM NaMES buffer at pH 7.0, 0.1mg/ml tRNA, 10% dextran sulfate (Pharmacia; molecular weight approximately 500,000), 1 picomole of the tailed ODS 81, 0.2 picomoles of the tailed ODS 56 and sample RNA (approximately $10^{-16}$ moles) in a volume of approximately 200 microliters was incubated for 10 minutes at approximately 58°C. One-tenth volume of 5.0 M LiCl was added, mixed and then drawn through the filter under gentle vacuum (approximately 0.2 inches Hg). This step took approximately 5-10 minutes.

The filter was washed (this is an optional step) under vacuum with 2 x 50 microliters of 2.4M tetraethylammonium chloride (TEA Cl)/0.5% tween 20. This was a stringency wash to remove improperly formed hybrids. The TEA Cl melts improper duplexes without melting the U:A bonds. The filter was then rinsed with 250 microliters of 0.1M NaCl and 0.1% SDS. Next, 100 microliters of 0.5 micrograms/ml streptavidin-HRP solution was passed through the filter. The filter was washed with 3 x 250 microliters of 0.1 M NaCl and 0.1% SDS. Then, a wash solution of 250 microliters of 0.1 M NaCl, pH 4.7 sodium citrate buffer was passed through the filter.

With the vacuum turned off, 30 microliters of a TMB/buffer/peroxide staining mixture was placed on top of the filter and allowed to sit (incubate at room temperature) for approximately 10 minutes. The reaction was stopped by sucking the TMB solution through the filter under 5-10 inches of mercury. Staining of the filter paper by the TMB indicated the presence of M. pneumoniae rRNA in the sample. The color change ranged from a very light green to a very dark green, depending on the amount of rRNA present in the sample. The hybridization mixture was quite viscous and passed through the filter slowly. All other washes or bindings (HRP) were over in much less than 1 minute.

The current detection limit of the assay is in the range of 100 attomoles ($10^{-16}$ moles) of rRNA or, phrased another way, it is capable of detecting as few as $10^8$ pieces of M. pneumoniae rRNA. The entire assay takes place in approximately 30 minutes, as there is no long hybridization step required.

The invention has been described herein with reference to certain preferred embodiments and Examples. However, since obvious variations will appear to those skilled in the art the invention is not to be considered limited thereto.

**Claims**

1. A method for determining the presence or amount of a target nucleic acid sequence in a sample suspected of containing said target, comprising:

a) providing a soluble capture oligonucleotide complementary to at least a portion of the target sequence and a soluble detectable oligonucleotide complementary to a substantially different portion of the target sequence, wherein the capture oligonucleotide has a homopolynucleotide tail covalently attached thereto;

b) mixing the capture oligonucleotide, detectable oligonucleotide and single stranded target nucleic acid in an aqueous solution under hybridizing conditions so as to form a soluble tripartite hybridization complex;

c) contacting said tripartite complex with an insoluble solid support having homopolynucleotide tails covalently attached thereto, wherein the immobilized tails are complementary to the tail on the capture oligonucleotide, and wherein the tails hybridize thereby immobilizing said tripartite complex; and

d) detecting or measuring the presence of the detectable oligonucleotide on said solid support.

2. The method of claim 1, wherein the capture and detectable oligonucleotides are at least 11 nucleotides long and optionally no more than 100 nucleotides long.

3. The method of claim 1 or claim 2, wherein the capture and detectable oligonucleotides are complementary to adjacent target sequences which either abut or are separated by one or more nucleotides.

4. The method of any of claims 1 to 3, wherein the detectable oligonucleotide has at least one radioactive, fluorescent, chemiluminescent or other reporter molecule covalently attached thereto, and/or the detectable oligonucleotide has at least one detectable ligand covalently attached thereto, the ligand optionally being biotin, an enzyme, an antibody-specific antigen or an antigen-specific antibody.

5. The method of any of the preceding claims, wherein the homopolynucleotide tails are poly A, poly G, poly C, poly T or poly U tails, the homopolynucleotide tails optionally being at least 50 nucleotides long.

6. The method of any one of the preceding claims, wherein prior to or simultaneously with step (b), the sample is treated so as to lyse cells containing the target nucleic acid and to provide target nucleic acid in single stranded form.

7. A synthetic nucleic acid probe having a sequence complementary to at least a portion of the RNA sequence:

5′    ACCGCAUAAGAACUUUGGUUCGCAUGAA
UCAAAGUUGAAAGGACCUGCAAGGGUUC
GUUAUUUGAUGAGGGUGCGCCAUAUCAG
CUAGUUGGUGGGGUAACGGCCUACCAAGGCA 3′

the probe optionally having at least ten bases and/or being tailed with from 1-150 additional nucleotides, the additional nucleotides not forming a sequence complementary to the RNA sequence.

8. The probe of Claim 20, wherein the probe sequence is:

3'      GTATTCTTGAAACCAAGCGTACTTA GTTTCAAC 5',
3'      CTGGACGTTCCCAAGCAATAAACTA CTCCCACGCGGT 5',
3' CCAAGCGTACTTAGTTTCAACTTTCC 5',
3' TGGACGTTCCCAAGCAATAAACTACT 5', or
3'      ATAGTCGATCAACCACCCCATTGCCGGATGGTTCCGT 5'.

9. In a method for identifying the presence of Mycoplasma pneumoniae 16S ribosomal RNA in a sample by using at least one nucleic acid probe complementary to the ribosomal RNA in a hybridization assay, the improvement comprising: providing at least one synthetic nucleic acid probe of Claim 7, the nucleic acid probe optionally being as further defined in Claim 8.

10. In a method for identifying the presence of Mycoplasma pneumoniae 16S ribosomal RNA in a sample by using two different nucleic acid probes complementary to the ribosomal RNA in a one-step sandwich hybridization assay, the improvement comprising: providing first and second synthetic nucleic acid probes of Claim 7, the nucleic acid probes optionally being as further defined in Claim 8.

11. A nucleic acid probe complementary to at least a portion of the RNA fragment from the 16S rRNA of M. pneumoniae having the nucleotide sequence:

5'      ACCGCAUAAGAACUUUGGUUCGCAUGAA UCAAAGUUGAAAGGACCUGCAAGGGUUC GUUAUUUGAUGAGGGUGCGCCAUAUCAG CUAGUUGGUGGGGUAACGGCCUACCAAGGCA 3'

Claims for the following Contracting State ES:

1. A method for determining the presence or amount of a target nucleic acid sequence in a sample suspected of containing said target, comprising:

a) providing a soluble capture oligonucleotide complementary to at least a portion of the target sequence and a soluble detectable oligonucleotide complementary to a substantially different portion of the target sequence, wherein the capture oligonucleotide has a homopolynucleotide tail covalently attached thereto;

b) mixing the capture oligonucleotide, detectable oligonucleotide and single stranded target nucleic acid in an aqueous solution under hybridizing conditions so as to form a soluble tripartite hybridization complex;

c) contacting said tripartite complex with an insoluble solid support having homopolynucleotide tails covalently attached thereto, wherein the immobilized tails are complementary to the tail on the capture oligonucleotide, and wherein the tails hybridize thereby immobilizing said tripartite complex; and

d) detecting or measuring the presence of the detectable oligonucleotide on said solid support.

2. The method of claim 1, wherein the capture and detectable oligonucleotides are at least 11 nucleotides long and optionally no more than 100 nucleotides long.

3. The method of claim 1 or claim 2, wherein the capture and detectable oligonucleotides are complementary to adjacent target sequences which either abut or are separated by one or more nucleotides.

4. The method of any of claims 1 to 3, wherein the detectable oligonucleotide has at least one radioactive, fluorescent, chemiluminescent or other reporter molecule covalently attached thereto, and/or the detectable oligonucleotide has at least one detectable ligand covalently attached thereto, the ligand optionally being biotin, an enzyme, an antibody-specific antigen or an antigen-specific antibody.

5. The method of any of the preceding claims, wherein the homopolynucleotide tails are poly A, poly G, poly C, poly T or poly U tails, the homopolynucleotide tails optionally being at least 50 nucleotides long.

6. The method of any one of the preceding claims, wherein prior to or simultaneously with step (b), the sample is treated so as to lyse cells containing the target nucleic acid and to provide target nucleic acid in single stranded form.

7. In a method for identifying the presence of Mycoplasma pneumoniae 16S ribosomal RNA in a sample by using at least one nucleic acid probe complementary to the ribosomal RNA in a hybridization assay, the improvement comprising: providing at least one synthetic nucleic acid probe having a sequence complementary to at least a portion of the RNA sequence:

5'      ACCGCAUAAGAACUUUGGUUCGCAUGAA UCAAAGUUGAAAGGACCUGCAAGGGUUC GUUAUUUGAUGAGGGUGCGCCAUAUCAG CUAGUUGGUGGGGUAACGGCCUACCAAGGCA 3'

or providing at least one nucleic acid probe complementary to at least a portion of the RNA fragment of the 16S rRNA of M. pneumoniae having the above nucleotide sequence.

8. In a method for identifying the presence of Mycoplasma pneumoniae 16S ribosomal RNA in a sample by using two different nucleic acid probes complementary to the ribosomal RNA in a one-step sandwich hybridization assay, the improvement comprising:
providing first and second nucleic acid probes as defined in Claim 7.

9. The method of Claim 29, wherein the first and second probes:

(a) have non-overlapping sequences,

(b) overlap but also have non-overlapping sequences,

(c) are adjacent sequences and wherein the sequences abut or are separated by one or more bases, or

(d) each have at least ten bases.

10. The method of Claim 8 or Claim 9, wherein one of the probes has a detectable label attached and the other probe is immobilizable or immobilized to a solid support.

11. The method of any of Claims 7 to 10, wherein the probe or one of the probes has the sequence:

3′ GTATTCTTGAAACCAAGCGTACTTA GTTTCAAC 5′,
3′ CTGGACGTTCCCAAGCAATAAACTA CTCCCACGCGGT 5′,
3′ CCAAGCGTACTTAGTTTCAACTTTCC 5′,
3′ TGGACGTTCCCAAGCAATAAACTACT 5′, or
3′ ATAGTCGATCAACCACCCCATTGCCG- GATGGTTCCGT 5′.

EP 0 305 145 A2

FIGURE 1

M. pneumoniae 16S rRNA and ODS 55/56 probe sequences

16S rRNA:
base 158 to
base 237

5' ACCGCAUAAGAACUUUGGUUCGCAUGAAUCAAAGUUGAAAGGACCUGCAAGGGUUCGUUAUUUGAUGAGGGUGCGCCAUA 3'
............................................ ......................................
3' GTATTCTTGAAACCAAGCGTACTTAGTTTCAAC          CTGGACGTTCCCAAGCAATAAACTACTCCCACGCGGT 5'
              ( ODS 55 )                                    ( ODS 56 )

# FIGURE 2

Mycoplasma pneumoniae 16S rRNA

(MPRDNA1)

```
    10         20         30         40         50
UUUCUGAGAG UUUGAUCCUG GCUCAGGAUU AACGCUGGCG GCAUGCCUAA

    60         70         80         90        100
UACAUGCAAG UCGAUCGAAA GUAGUAAUAC UUUAGAGGCG AACGGGUGAG

   110        120        130        140        150
UAACACGUAU CCAAUCUACC UUAUAAUGGG GGAUAACUAG UUGAAAGACU

   160        170        180        190        200
AGCUAAUACC GCAUAAGAAC UUUGGUUCGC AUGAAUCAAA GUUGAAAGGA

   210        220        230        240        250
CCUGCAAGGG UUCGUUAUUU GAUGAGGGUG CGCCAUAUCA GCUAGUUGGU

   260        270        280        290        300
GGGGUAACGG CCUACCAAGG CAAUUACGUG UACUGUGCUG AGAAGUAGAA
```

UAGCCAC .